# FASCICULE DE BREVET EUROPEEN

(11) **EP 1 877 561 B1**
(45) Date de publication et mention de la délivrance du brevet: **13.03.2013**
(21) Numéro de dépôt: 06764594.5
(22) Date de dépôt: 03.05.2006
(51) Int. Cl.: C12N 15/56, C12N 9/24, A23K 1/165

(54) **GENE abfB-1 DE PENICILLIUM FUNICULOSUM**
ABFB-1-GEN AUS PENICILLIUM FUNICULOSUM
ABFB-1 GENE OF PENICILLIUM FUNICULOSUM

(30) Priorité: 04.05.2005 FR 0504562
(43) Date de publication de la demande: 16.01.2008
(73) Titulaire: Adisseo France S.A.S., 92160 Antony (FR)
(72) Inventeur: FRANCOIS, Jean Marie, F-31320 Castenet-Tolosan (FR); PARROU, Jean-Luc, F-31400 Toulouse (FR); TOURRASSE, Olivier, F-31100 Toulouse (FR); NORE, Olivier, F-37210 Vernou sur Brenne (FR)
(74) Mandataire: Jouannic, Nathalie
(86) Numéro de dépôt international: PCT/FR2006/000998
(87) Numéro de publication internationale: WO 2006/117482

(56) Documents cités:
- WO-A-99/57325
- WO-A-2004/018662
- CARVALLO MARCELA ET AL: "Characterization of an alpha-L-arabinofuranosidase gene (abf1) from Penicillium purpurogenum and its expression." MYCOLOGICAL RESEARCH, vol. 107, no. 4, avril 2003 (2003-04), pages 388-394, XP009059573 ISSN: 0953-7562 cité dans la demande
- DE IOANNES PABLO ET AL: "An alpha-L-arabinofuranosidase from Penicillium purpurogenum: Production, purification and properties" JOURNAL OF BIOTECHNOLOGY, vol. 76, no. 2-3, 21 janvier 2000 (2000-01-21), pages 253-258, XP002395553 ISSN: 0168-1656
- PARK NYUN HO ET AL: "A new method for the preparation of crystalline L-arabinose from arabinoxylan by enzymatic hydrolysis and selective fermentation with yeast" BIOTECHNOLOGY LETTERS, vol. 23, no. 5, mars 2001 (2001-03), pages 411-416, XP002361552 ISSN: 0141-5492
- CLINCHE LE F ET AL: "ALPHA-L-ARABINOFURANOSIDASES FROM ASPERGILLUS TERREUS WITH POTENTIAL APPLICATION IN ENOLOGY: INDUCTION, PURIFICATION, AND CHARACTERIZATION" JOURNAL OF AGRICULTURAL AND FOOD CHEMISTRY, AMERICAN CHEMICAL SOCIETY. WASHINGTON, US, vol. 45, no. 7, juillet 1997 (1997-07), pages 2379-2383, XP000656899 ISSN: 0021-8561 cité dans la demande
- DATABASE EMBL 14 novembre 2001 (2001-11-14), HASHIMOTO T ET AL: "Alpha-L-arabinofuranosidase of Aspergillus oryzae HL15" XP002395261 Database accession no. AB073860
- DATABASE UniProt 1 décembre 2001 (2001-12-01), HASHIMOTO ET AL: "Alpha-L-arabinofuranosidase B" XP002395262 Database accession no. Q96VA1
- MARGOLLES-CLARK E ET AL: "CLONING OF GENES ENCODING ALPHA-L-ARABINOFURANOSIDASE AND BETA-XYLOSIDASE FROM TRICHODERMA REESEI BY EXPRESSION IN SACCHAROMYCES CEREVISIAE" APPLIED AND ENVIRONMENTAL MICROBIOLOGY, WASHINGTON,DC, US, vol. 62, no. 10, octobre 1996 (1996-10), pages 3840-3846, XP000900963 ISSN: 0099-2240 cité dans la demande

## Description

L'invention concerne le gène *abfB-1* isolé de *Penicillium funiculosum* et le polypeptide ABFB-1 codé par ce gène ayant une activité α-L-arabinofuranosidase B.

*Penicillium funiculosum* est un Talaromyces appartenant à la famille des *Aspergilleae.* L'isolement de ce microorganisme à partir de nombreux substrats organiques sujets à une contamination aérienne ou aqueuse, indique que ce champignon possède une panoplie d'enzymes hydrolytiques d'une richesse surprenante. L'utilisation de ce cocktail enzymatique dans l'alimentation animale contribue à la dépolymérisation des substances organiques naturelles et permet d'améliorer leur digestibilité. WO 99/57325 décrit ainsi une souche de *Penicillium funiculosum* dénommée IMI378536 produisant un mélange d'enzymes particulièrement adapté à l'alimentation animale. Néanmoins les cocktails enzymatiques produits par *Penicillium funiculosum* sont peu caractérisés biochimiquement. En effet seul un nombre restreint d'activités enzymatiques comme les xylanases, les β-glucanases sont en général mesurées sur les moûts de fermentation obtenus. Ces activités ne reflètent qu'une fraction de la population enzymatique présente dans le cocktail.

Les composés hémi-cellulolytiques issus de l'agriculture constituent la deuxième réserve de polysaccharides après la cellulose au sein des tissus végétaux. Ce groupe est caractérisé par une large variété d'hétéropolysaccharides, dont les principaux représentants sont les xylans, les arabinanes, les galactanes, les glucanes, et les mannanes. L'arabinose sous sa forme furfural est largement représenté au sein des hétéropolysaccharides tels que les arabinanes et les arabinoxylanes. L'arabinane est un polymère de résidus arabinofuranose liés par des liaisons α-1-5 et il peut être substitué par 1 ou 2 résidus arabinose en position O-2 ou O-3. En ce qui concerne les arabinoxylanes, les résidus α-L-arabinofuranosyl sont liés sur la chaîne principale β-1-4-xytopyranosyt par liaisons α-1-3 et α-1-2. La présence de résidus arabinose sur ces chaînes latérales peut restreindre l'hydrolyse enzymatique des composés hémi-cellulolytiques dans de nombreuses applications industrielles telles que l'amélioration de la digestibilité de l'alimentation animale. Les enzymes clivant les liaisons α-L-arabinofuranosidiques peuvent agir synergiquement avec les xylanases pour permettre l'hydrolyse des arabinoxylanes et arabinanes.

Les activités arabinases (endo-, exo-arabinases et majoritairement les activités α-L-arabinofuranosidases) peuvent donc contribuer activement et de façon synergique avec les xylanases à la dépolymérisation des composés hemi-cellulolytiques. Les composés hemi-cellulolytiques et pectiques peuvent représenter jusqu'à 50% des carbohydrates totaux présents dans les plantes et ils constituent une importante source d'énergie pour les animaux. L'amélioration de la digestibilité de ces composés est corrélée avec la diminution du degré de substitution des résidus arabinosyls au sein des composés hémi-cellulolytiques (Brice, R.E., Morrison, I.M. 1982, Carbohydr. Res. 101 : 93-100).

Les enzymes hydrolysant les liaisons entre des résidus L-arabinose ont été isolées à partir de micro-organismes tels que les bactéries ou les champignons filamenteux. Les arabinosidases sont constituées principalement d'α-L-arabinoturanosidases (EC 3.2.1.55) qui sont capables d'hydrolyser les résidus α-L-arabinofuranosyl non réducteurs issus du L-arabinoxylane ou de composés tels que les arabinanes, et les arabinogalactanes.

Les α-L-arabinofuranosidases (EC 3.2.1.55) ont été classées en deux familles de Glycosides Hydrolases (GH 51 et GH 54) selon leur similarités de séquence protéique. Ces deux familles diffèrent de par leur spécificité de substrat contenu dans les polysaccharides. Le premier groupe (GH 51) contient les arabinofuranosidases de type A qui agissent seulement sur de petites structures linéaires d'arabinofuranosyl oligosaccharides liés en α-1-5. Le second groupe est constitué d'arabinofuranosidases de type B (GH 54) qui catalysent l'hydrolyse des liaisons α-1,5 ; α-1,3 et α-1,2 des chaînes latérales contenues dans les composés arabinofuranosyl-oligosaccharides.

Les arabinofuranosidases B (ABFB) ont été isolées à partir de nombreuses bactéries mais également à partir de champignons filamenteux. Le genre *Aspergillus* est le plus représenté, mais elles ont été également isolées à partir des genres *Trichoderma, Penicillium, et Fusarium.*

WO96/29416 WO96/06935, WO 2004/018662 et US 5,989,887 décrivent des gènes d'arabinofuranosidase d'*Aspergillus niger.* L'alignement de séquences protéiques indique que la protéine abfB de *A. niger* est à 72,4% identique à la protéine ABFB-1 de *P. funiculosum.* Aucune des caractéristiques essentielles à l'utilisation du polypeptide en nutrition animale n'est décrite dans ces demandes.

Clinche et al. (J. Agric. Food Chem., 45, 2379-2383, 1997) ont décrit trois -L-arabinofuranosidases issues de *Aspergillus terreus* ayant une application potentielle en oenologie.

Gielkens et al. (Microbiology, 145, 735-741, 1999) ont décrit le gène *abfB d'Aspergillus nidulans.*

Les gènes *abfB d'Aspergillus kawachii* et *d'Aspergillus awamori* ont été décrits par Koseki et al. (J. of Bioscience and Bioengineering, vol. 96, no. 3, 232-241, 2003). Ces enzymes ont des applications dans la fermentation de la liqueur japonaise *shochu.*

Le gène *abfB* du champignon filamenteux *Trichoderma reesei* a été décrit par Margolles-Clark et al. (Applied and Environmental Microbiology, 3840-3846, 1996).

Panagiotou *et al.* ont également décrits deux alpha-L-arabinofuranosidases extracellulaires issues de *Fusarium oxysporum.* (Can J Microbiol. 2003 : 49(10):639-4).

Carvallo et al. (Mycol. Res., 107 (4), 388-394, 2003) ont décrit l'α-L-arabinofuranosidase B de *Penicillium purpurogenum.* L'alignement de séquences protéiques indique que la protéine abf-1 de *P. purpurogenum* est à 85,6% identique à la protéine ABFB-1 de *P. funiculosum.* Aucune des caractéristiques essentielles à l'utilisation du polypeptide en nutrition animale n'est décrite dans cet article.

Sakamoto et al. (FEBS Letters 560, 199-204, 2004) ont décrit le gène *abnx* de *Penicillium chrysogenum* codant cependant pour une activité arabinanase distincte de l'activité des ABFB.

Cependant, ces enzymes ABFB n'ont pas les qualités optimales requises pour une application en alimentation animale. En effet, pour être utilisables en alimentation animale, les ABFB doivent posséder des propriétés compatibles avec les traitements que subissent les aliments destinés à cette alimentation. En particulier, l'activité des enzymes utilisées doit être stable dans les conditions de température et de pH des procédés, et si possible être optimale dans la préparation de ces aliments ainsi que dans les conditions présentes dans le système digestif des animaux ingérant ces aliments.

De plus, ces enzymes doivent avoir un large spectre d'action (débranchement) sur les hétéropolysaccharides (arabinanes, arabinoxylanes et arabinogalactanes) pour permettre une amélioration efficace de la digestibilité des aliments par les animaux. Cette amélioration de la digestibilité des aliments pour animaux permet d'augmenter leur valeur nutritionnelle. Ainsi, les enzymes ayant une spécificité (stéréo-spécificité, énantiosélectivité), une activité ou une affinité améliorée envers les substrats naturels arabinoxylanes et arabinanes présentent un grand intérêt pour l'alimentation animale.

La présente invention décrit une L-arabinofuranosidase B (ABFB-1) de *Penicillium funiculosum,* adaptée pour une application dans la nutrition animale ainsi que le gène codant pour cette enzyme. L'invention concerne aussi les homologues, les variants et les fragments de l'ABFB-1 conservant les mêmes propriétés catalytiques.

Avantageusement, les enzymes ABFB selon l'invention présentent une température optimale élevée.

Un autre avantage de la présente invention est que l'expression de l'ABFB-1 de *Penicillium funiculosum* est naturellement fortement induite chez ce champignon dans des conditions d'induction d'enzymes cellulolytiques et hémi-cellulolytiques (milieu de culture de type industriel pour la production d'enzymes cellulolytiques et hémi-cellulolytiques).

Les enzymes selon l'invention ont également d'autres applications industrielles ou agro-industrielles. On citera notamment le traitement des jus de fruits, la fabrication du papier, la conversion de biomasses hemicellulolytiques en carburants ou produits chimiques, la préparation de boissons alcoolisées par fermentation.

### Description des séquences

SEQ ID No. 1 : Séquence génomique du gène *abfB-1* de *Penicillium funiculosum.*
SEQ ID No. 2 : Séquence du polypeptide ABFB-1 de *Penicillium funiculosum* ayant une activité α-L-arabinofuranosidase de type B.
SEQ ID No. 3 : Amorce *XbaI-abfB.*
SEQ ID No. 4 : Amorce HindIII-*abfB*.

### Description de l'invention

La présente invention concerne un polypeptide adapté à l'utilisation en nutrition animale comprenant un polypeptide choisi parmi les polypeptides suivants:
- le polypeptide de la SEQ ID No. 2 ,
- le polypeptide dont la séquence est comprise entre la position 28 et la position 507 de la SEQ ID No. 2,

L'invention concerne aussi un polynucléotide, codant pour une une activité α-L-arabinofuranosidase B, choisi parmi les polynucléotides suivants :
- le polynucléotide dont la séquence est comprise entre la position 845 et la position 2368 de la SEQ ID No. 1,
- le polynucléotide dont la séquence est comprise entre la position 926 et la position 2368 de la SEQ ID No. 1

Un autre objet de la présente invention est un polynucléotide ayant la séquence représentée à la SEQ ID No. 1 ou la séquence complémentaire à la SEQ ID No. 1.

L'invention se rapporte aussi à des cassettes d'expression comprenant dans le sens de la transcription:
- un promoteur fonctionnel dans un organisme hôte;
- un polynucléotide selon l'invention; et
- une séquence terminatrice fonctionnelle dans le même organisme hôte.

Un autre objet de l'invention est un vecteur comprenant un polynucléotide selon l'invention et/ou une cassette d'expression selon l'invention.

L'invention concerne aussi un organisme hôte transformé avec un polynucléotide selon l'invention, une cassette d'expression selon l'invention et/ou un vecteur selon l'invention.

Dans un mode de réalisation de l'invention, l'organisme hôte est choisi parmi les levures et les champignons filamenteux.

De préférence, l'organisme hôte est une souche de *Penicillium funiculosum.*

L'invention se rapporte également à un additif nutritionnel pour animaux comprenant un polypeptide selon l'invention, un organisme hôte selon l'invention ou un moût de fermentation d'un organisme hôte selon l'invention.

Préférentiellement, cet additif nutritionnel se présente sous forme liquide ou sous forme de poudre.

Un autre aspect de l'invention est un aliment pour animaux comprenant une base nutritionnelle pour animaux et un additif nutritionnel pour animaux selon l'invention.

L'invention concerne aussi l'utilisation d'un polypeptide ABFB selon l'invention ou d'un organisme hôte selon l'invention pour la fabrication d'un additif nutritionnel pour animaux ou d'un aliment pour animaux.

Un autre objet de l'invention est l'utilisation d'un polypeptide ABFB selon l'invention ou d'un organisme hôte selon l'invention pour l'hydrolyse des liaisons α-L-arabinofuranosyl des composés arabinofuranosyl-oligosaccharides.

### Polypeptides

La présente invention concerne donc des polypeptides ABFB ayant une activité α-L-arabinofuranosidase B. De préférence, ces polypetides sont isolés de *Penicillium funiculosum.*

On entend par "α-L-arabinofuranosidase B", des α-L-arabinofuranosidases (EC 3.2.1.55) de type B (GH 54) qui catalysent l'hydrolyse des liaisons α-1,5 ; α-1,3 et α-1,2 des chaînes latérales contenues dans les composés arabinofuranosyl-oligosaccharides.

L'α-L-arabinofuranosidase B de la souche IMI378536 de *Penicillium funiculosum* est représentée à la SEQ ID No.2.

On entend par "polypeptide adapté à l'utilisation en nutrition animale", un polypeptide dont les caractéristiques sont telles qu'il convient pour la nutrition animale. Les caractéristiques essentielles à une utilisation en nutrition animale sont notamment le pH et la température auxquels l'enzyme est active. En effet, le pH du système digestif des animaux est acide et il est donc essentiel que l'enzyme reste active à ce pH, ce afin de conserver son activité dans l'hydrolyse des résidus L-arabinose. En outre, la mise en forme de l'enzyme dans un additif nutritionnel ou dans l'aliment de l'animal implique des traitements et une température supérieure à la température ambiante. L'activité des enzymes utilisées doit donc être stable dans les conditions des procédés, notamment les conditions de températures.

Selon un mode de réalisation de la présente invention, le polypeptide présente une activité α-L-arabinofuranosidase B à un pH acide, par exemple inférieur à 5, de préférence inférieur à 4. Egalement, selon un mode de réalisation de la présente invention, le polypeptide présente une activité α-L-arabinofuranosidase B optimale entre pH 2 et pH 3,5.

Selon un mode de réalisation préférée de la présente invention, le polypetide présente une activité α-L-arabinofuranosidase B à des températures supérieures à la température ambiante. De préférence, le polypeptide de la présente invention possède une activité α-L-arabinofuranosidase B optimale à une température comprise entre 40°C et 70°C, plus préférentiellement entre 50°C et 65°C.

Dans un mode de réalisation préféré, les polypeptides selon l'invention sont glycosylés. Le polypeptide de la SEQ ID No. 2 possède notamment des sites de N-glycosylation à l'acide aminé 92 et à l'acide aminé 376 . Dans un mode de réalisation préféré, les résidus asparagine en position 92 et 376 du polypeptide de la SEQ ID No. 2 sont glycosylés.

L'α-L-arabinofuranosidase B de *Penicillium funiculosum* est une enzyme secrétée par le champignon dans son environnement extracellulaire. Le polypeptide de la SEQ ID No. 2 comprend ainsi un peptide signal de 27 acides aminés. L'invention a également pour objet le polypeptide mature obtenu après clivage du peptide signal. En particulier, l'invention se rapporte au polypeptide dont la séquence est comprise entre la position 28 et la position 507 de la SEQ ID No. 2.
Dans un autre mode de réalisation, le peptide signal du polypeptide de la SEQ ID No. 2 peut être remplacé par un peptide signal hétérologue pour l'expression et la secrétion du polypeptide de la SEQ ID No. 2 par un organisme hôte hétérologue.

L'invention se rapporte également à des fragments du polypeptide de la SEQ ID No. 2 ayant une activité α-L-arabinofuranosidase B.

Le terme "fragment" d'un polypeptide désigne un polypeptide comprenant une partie mais pas la totalité du polypeptide dont il est dérivé.

Ce fragment du polypeptide de la SEQ ID No. 2 conserve son activité α-L-arabinofuranosidase B. L'invention concerne donc les fragments biologiquement actifs du polypeptide de la SEQ ID No. 2. Le terme "fragment biologiquement actif" désigne un fragment d'un polypeptide conservant la fontion du polypeptide dont il est dérivé. Les fragments biologiquement actifs du polypeptide de la SEQ ID No. 2 conservent ainsi la fonction du polypeptide ABFB-1 de *Penicillium funiculosum.* Ces fragments biologiquement actifs ont une activité de α-L-arabinofuranosidase B.

Les méthodes de préparation de fragments d'un polypeptide ainsi que les techniques de mesure de l'activité α-L-arabinofuranosidase **B** sont bien connues de l'homme du métier.

Les polypeptides selon l'invention sont isolés ou purifiés de leur environnement naturel. Les polypeptides peuvent être préparés au moyen de différents procédés. Ces procédés sont notamment la purification à partir de sources naturelles telles que des cellules exprimant naturellement ces polypeptides, la production de polypeptides recombinants par des cellules hôtes appropriées et leur purification ultérieure, la production par synthèse chimique ou, enfin, une combinaison de ces différentes approches. Ces différents procédés de production sont bien connus de l'homme du métier. Ainsi, les polypeptides ABFB de la présente invention peuvent être isolés à partir de *Penicillium funiculosum.* Dans un autre mode de réalisation, les polypeptides ABFB de la présente invention sont isolés à partir d'organisme hôtes recombinants exprimant un polypeptide ABFB selon l'invention.

L'invention a également pour objet des protéines de fusion, des protéines recombinantes ou des protéines chimères comprenant les polypeptides selon l'invention. Le terme "polypeptide" désigne également des protéines ainsi que des polypeptides modifiés.

Les polypeptides selon l'invention ont une activité ABFB et conservent de préférence les propriétés catalytiques de l'enzyme ABFB-1 de *Penicillium funiculosum.* En particulier, ces polypeptides présentent une activité optimale à 60°C et à pH 3.4.

### Polynucléotides

L'invention concerne aussi des polynucléotides codant pour une activité α-L-arabinofuranosidase B. De préférence, ces polynucléotides codent pour une α-L-arabinofuranosidase B de *Penicillium funiculosum.*

Selon la présente invention, on entend par "polynucléotide " une chaine nucléotidique simple brin ou son complémentaire pouvant être de type ADN ou ARN, ou une chaine nucléotidique double brin pouvant être de type ADN complémentaire ou génomique. De préférence, les polynucléotides de l'invention sont de type ADN, notamment d'ADN double brin. Le terme "polynucléotide" désigne également les polynucléotides modifiés.

Les polynucléotides de la présente invention sont isolés ou purifiés de leur environnement naturel. De préférence, les polynucléotides de la présente invention peuvent être préparés par les techniques classiques de biologie moléculaire telles que décrites par Sambrook et al. (Molecular Cloning : A Labratory Manual, 1989) ou par synthèse chimique.

Dans un premier mode de réalisation, l'invention se rapporte au polynucléotide dont la séquence est comprise entre la position 845 et la position 2368 de la SEQ ID No. 1. Ce polynucléotide code pour l'enzyme ABFB-1 de *Penicillium funiculosum* de la SEQ ID No. 2.

Dans un deuxième mode de réalisation, l'invention concerne le polynucléotide dont la séquence est comprise entre la position 926 et la position 2368 de la SEQ ID No. 1. Ce polynucléotide code pour le polypeptide mature ABFB de *Penicillium funiculosum* après clivage du peptide signal.

L'invention concerne aussi des polynucléotides capables de s'hybrider de manière sélective avec le polynucléotide dont la séquence est comprise entre la position 845 et la position 2368 de la SEQ ID No. 1 et/ou avec le polynucléotide dont la séquence est comprise entre la position 926 et la position 2368 de la SEQ ID No. 1. De préférence, l'hybridation sélective est effectuée dans des conditions de moyenne stringence et préférentiellement dans des conditions de forte stringence. Ces polynucléotides codent pour une activité α-L-arabinofuranosidase B. De préférence, ces polynucléotides codent pour une α-L-arabinofuranosidase B de *Penicillium funiculosum.*

Par "séquence capable de s'hybrider de manière sélective", on entend selon l'invention les séquences qui s'hybrident avec la séquence de référence à un niveau supérieur au bruit de fond de manière significative. Le niveau du signal généré par l'interaction entre la séquence capable de s'hybrider de manière sélective et les séquences de référence est généralement 10 fois, de préférence 100 fois plus intense que celui de l'interaction des autres séquences d'ADN générant le bruit de fond. Les conditions d'hybridation stringentes permettant une hybridation sélective sont bien connues de l'homme du métier. En général la température d'hybridation et de lavage est inférieure d'au moins 5°C au Tm de la séquence de référence à un pH donné et pour une force ionique donnée. Typiquement la température d'hybridation est d'au moins 30°C pour un polynucléotide de 15 à 50 nucléotides et d'au moins 60°C pour un polynucléotide de plus de 50 nucléotides. A titre d'exemple, l'hybridation est réalisée dans le tampon suivant: 6X SSC, 50 mM Tris-HCl (pH 7.5), 1 mM EDTA, 0.02% PVP, 0.02% Ficoll, 0.02% BSA, 500 µg/ml sperme de saumon dénaturé DNA. Les lavages sont par exemple réalisés successivement à faible stringence dans un tampon 2X SSC, 0,1% SDS, à moyenne stringence dans un tampon 0,5X SSC, 01% SDS et à forte stringence dans un tampon 0,1X SSC, 0,1 %SDS. L'hybridation peut bien entendu être effectuée selon d'autres méthodes usuelles bien connues de l'homme du métier (voir notamment Sambrook et al., Molecular Cloning : A Labratory Manual, 1989). De préférence, les polynucléotides s'hybridant de manière sélective à un polynucléotide de référence conservent la fonction de la séquence de référence. Dans le cas présent, les polynucléotides, s'hybridant de manière sélective avec le polynucléotide dont la séquence est comprise entre la position 845 et la position 2368 de la SEQ ID No. 1 et/ou avec le polynucléotide dont la séquence est comprise entre la position 926 et la position 2368 de la SEQ ID No. 1, codent pour une activité α-L-arabinofuranosidase B.

L'invention se rapporte de manière générale aux polynucléotides codant pour les polypeptides selon l'invention. En raison de la dégénerescence du code génétique, différents polynucléotides peuvent coder pour un même polypeptide.

Un autre objet de la présente invention est un polynucléotide dont la séquence est représentée à la SEQ ID No. 1. Le polynucléotide de la SEQ ID No. 1 comprend des séquences flanquant le cadre ouvert de lecture (ORF) du gène *abfB-1* de *Penicillium funiculosum.* Il s'agit notamment des séquences promotrices et terminatrices du gène *abfB-1.* Le gène *abfB* peut être exprimé à partir de ses séquences régulatrices homologues notamment pour une surexpression dans *Penicillium funiculosum* ou dans d'autres champignons filamenteux.

Dans un autre mode de réalisation, le gène abfB peut être exprimé dans différents organismes hôtes tels que les bactéries, les levures et les champignons par exemple. Le gène *abfB* peut être exprimé dans un organisme hôte sous le contrôle du promoteur de la SEQ ID No. 1 de la présente invention ou sous le contrôle d'un promoteur hétérologue.

### Cassettes d'expression

Selon un mode de réalisation de l'invention, un polynucléotide codant pour un polypeptide selon l'invention est inséré dans une cassette d'expression en utilisant des techniques de clonage bien connues de l'homme du métier. Cette cassette d'expression comprend les éléments nécessaires à la transcription et à la traduction des séquences codant pour les polypeptides selon l'invention.

Avantageusement, cette cassette d'expression comprend à la fois des éléments permettant de faire produire un polypeptide par une cellule hôte et des éléments nécessaires à la régulation de cette expression.

Ces cassettes d'expression comprennent dans le sens de la transcription:
- un promoteur fonctionnel dans un organisme hôte;
- un polynucléotide selon l'invention;
- une séquence terminatrice fonctionnelle dans le même organisme hôte.

Tout type de séquence promotrice peut être utilisée dans les cassettes d'expression selon l'invention. Le choix du promoteur dépendra notamment de l'organisme hôte choisi pour l'expression du gène d'intérêt. Certains promoteurs permettent une expression constitutive alors que d'autres promoteurs sont au contraire inductibles. Parmi les promoteurs fonctionnels dans les champignons, on citera notamment celui de glyceraldehyde-3-phosphate deshydrogenase d'*Aspergillus nidulans* (Roberts et al., Current Genet. 15:177-180, 1989). Parmi les promoteurs fonctionnels dans les bactéries, on citera notamment celui de la RNA polymérase du bacteriophage T7 (Studier et al., Methods in enzymology 185:60-89, 1990). Parmi les promoteurs fonctionnels dans les levures, on citera le promoteur du gène *GAL 1* (Elledge et al., Proc Natl Acad Sciences, USA. 88:1731-1735, 1991) ou les promoteurs *GAL4* et *ADH* de *S.cerevisiae.* Tous ces promoteurs sont décrits dans la littérature et bien connus de l'homme du métier.

Pour l'expression dans *Penicillium funiculosum,* on choisira par exemple des cassettes d'expression comprenant un promoteur histone H4.B, un promoteur acide aspartyl protéase ou un promoteur csl1 3 (WO 00/6.8401).

Les cassettes d'expression, selon la présente invention, peuvent en outre inclure toute autre séquence nécessaire à l'expression des polypeptides ou des polynucléotides comme par exemple des éléments de régulation ou des séquences signal permettant la sécrétion des polypeptides produits par l'organisme hôte. On peut notamment utiliser toute séquence de régulation permettant d'augmenter le niveau d'expression de la séquence codante insérée dans la cassette d'expression. Selon l'invention, on peut notamment utiliser, en association avec la séquence de régulation promotrice, d'autres séquences de régulation, qui sont situées entre le promoteur et la séquence codante, telles que des activateurs de transcription ("enhancer").

Une grande variété de séquences terminatrices sont utilisables dans les cassettes d'expression selon l'invention, ces séquences permettent la terminaison de la transcription et la polyadénylation de l'ARNm. Toute séquence terminatrice fonctionnelle dans l'organisme hôte sélectionné peut être utilisée.

Pour l'expression dans *Penicillium funiculosum,* on choisira par exemple des cassettes d'expression comprenant un terminateur histone H4.B, un terminateur acide aspartyl protease ou un terminateur csl13 (WO 00/68401).

La présente invention a également pour objet un polynucléotide comprenant une cassette d'expression selon l'invention, avantageusement les cassettes d'expression selon la présente invention sont insérées dans un vecteur.

### Vecteurs

La présente invention concerne donc également des vecteurs de réplication ou d'expression pour la transformation d'un organisme hôte comprenant au moins un polynucléotide ou une cassette d'expression selon la présente invention. Ce vecteur peut notamment correspondre à un plasmide, un cosmide, un bactériophage ou un virus dans lequel est inséré un polynucléotide ou une cassette d'expression selon l'invention. Les techniques de construction de ces vecteurs et d'insertion d'un polynucléotide de l'invention dans ces vecteurs sont bien connues de l'homme du métier. De manière générale, tout vecteur capable de se maintenir, de s'autorépliquer ou de se propager dans une cellule hôte afin d'induire notamment l'expression d'un polynucléotide ou d'un polypeptide peut être utilisé. L'homme du métier choisira les vecteurs appropriés en fonction de l'organisme hôte à transformer, et en fonction de la technique de transformation mise en oeuvre.

Les vecteurs de la présente invention sont notamment utilisés pour transformer un organisme hôte en vue de la réplication du vecteur et/ou de l'expression d'un polypeptide selon l'invention dans l'organisme hôte.

L'invention concerne aussi une méthode pour préparer un polypeptide selon l'invention comprenant les étapes suivantes:
- on transforme un organisme hôte avec un vecteur d'expression comprenant une cassette d'expression selon l'invention et/ou avec un polynucléotide selon l'invention,
- on isole les polypeptides produits par l'organisme hôte.

### Organismes hôtes

La présente invention a également pour objet, un procédé de transformation d'un organisme hôte par intégration dans leudit organisme hôte d'au moins un polynucléotide ou d'une cassette d'expression ou d'un vecteur selon l'invention. Le polynucléotide peut être intégré dans le génome de l'organisme hôte ou se repliquer de manière stable dans l'organisme hôte. Les méthodes de transformation des organismes hôtes sont bien connus de l'homme du métier et largement décrits dans la littérature.

La présente invention concerne également un organisme hôte transformé avec un polynucléotide, une cassette d'expression ou un vecteur selon l'invention. Par organisme hôte, on entend en particulier selon l'invention tout organisme mono ou pluricellulaire, inférieur ou supérieur, en particulier choisi parmi les bactéries, les levures et les les champignons. Par organisme hôte on entend un oorganisme non humain. De manière avantageuse, les levures sont choisies parmi *Pichia pastoris, Saccharomyces cerevisae, Yarrowia lipolytica* et *Schwanniomyces occidentalis.* Les champignons sont choisis parmi les *Aspergillus* et les *Penicilliums,* préférentiellement parmi *Penicillium funiculosum, Trichoderma reesei, Aspergillus niger, Aspergillus awamori, Aspergillus kawachii et Trichoderma koningii.* Dans un mode de réalisation préféré, l'organisme hôte est une souche de *Penicillium funiculosum* dans laquelle on exprime ou sur-exprime un polypeptide ABFB selon l'invention.

Les techniques de construction de vecteurs, de transformation d'organismes hôtes et d'expression de protéines hétérologues dans ces organismes sont largement décrites dans la littérature (Ausubel F.M. et al., "Current Protocols in Molecular Biology" Volumes 1 et 2, Greene Publishing Associates et Wiley -Interscience, 1989; T.Maniatis, E.F.Fritsch, J.Sambrook, Molecular Cloning A laboratory Handbook, 1982).

### Additifs alimentaires et aliments pour animaux

La présente invention concerne donc des additifs alimentaires apportant une activité α-L-arabinofuranosidase B. L'apport de ce type d'activité enzymatique permet d'améliorer la digestibilité de l'aliment et d'augmenter sa valeur nutritionnelle.

On entend par additif nutritionnel une substance ajoutée intentionnellement à un aliment, généralement en petites quantités, pour améliorer ses caractéristiques nutritionnelles ou sa digestibilité. Les additifs nutritionnels pour animaux peuvent par exemple contenir des vitamines, des sels minéraux, des acides aminés et des enzymes.

Typiquement, les additifs nutritionnels pour animaux comprennent un polypeptide selon l'invention, un organisme hôte selon l'invention où un moût de fermentation d'un organisme hôte selon l'invention. Ainsi, les polypeptides ayant une activité α-L-arabinofuranosidase B selon l'invention peuvent être purifiés ou isolés d'une souche de *Penicillium funiulosum* ou d'un organisme hôte recombinant pour la fabrication d'un additif nutritionnel pour animaux. Alternativement, une souche de *Penicillium funiculosum* ou un organisme hôte produisant des polypeptides AbfB peuvent être utilisés directement pour la fabrication d'un additif nutritionnel pour animaux. Dans un mode de réalisation préféré de l'invention, le surnageant de culture ou moût de fermentation d'une souche de *Penicillium funiculosum* ou d'un organisme hôte selon l'invention est utilisé pour la fabrication d'additifs nutritionnels pour animaux. Ce mode de réalisation est particulièrement avantageux lorsque les polypeptides ABFB sont secrétés par la souche de *Penicillium funiculosum* ou l' organisme hôte. Habituellement, ce surnageant de culture est concentré ou lyophilisé pour la fabrication de l'additif nutritionnel.

Ainsi, l'invention concerne aussi un procédé de préparation d'enzyme ABFB comprenant les étapes suivantes :
a) mise en culture d'une souche de *Penicillium funiculosum* ou d'un organisme hôte transformé selon l'invention dans des conditions d'induction de l'expression des ABFB,
b) séparation du surnageant de culture comprenant l'enzyme ABFB.

Ce surnageant de culture ou moût de fermentation peut ensuite être concentré ou lyophilisé pour la formulation d'un additif alimentaire ou d'un aliment pour animaux.

Si l'organisme hôte ne secrète pas l'enzyme ABFB dans le milieu de culture, une étape supplémentaire de cassage des cellules et de purification de l'extrait cellulaire peut être nécessaire.

Les additifs nutritionnels de la présente invention comprennent une activité α-L-arabinofuranosidase B mais peuvent également comprendre d'autres substances nutritionnelles comme des vitamines, des acides aminés ou des sels minéraux.

Les additifs selon l'invention accroissent la digestibilité des aliments, contribuant ainsi à une meilleure valorisation nutritionnelle des régimes à base de céréales (blé, orge, maïs, avoine, seigle, ...) et de tourteaux oléagineux (soja, tournesol, colza, ...) notamment.

La présente invention concerne aussi les aliments pour animaux comprenant une base nutritionnelle et un additif nutritionnel selon l'invention. Ces aliments se présentent habituellement sous la forme de farines ou de granulés dans lesquels sont incorporés les additifs selon l'invention.

On entend par aliment tout ce qui peut servir à la nourriture des animaux.

Les aliments pour animaux comprennent un polypeptide selon l'invention, un organisme hôte selon l'invention où un moût de fermentation d'un organisme hôte selon l'invention.

Pour l'élevage intensif des animaux, ces aliments comprennent habituellement une base nutritionnelle et des additifs nutritionnels.

On entend par base nutritionnelle, ce qui constitue l'essentiel de la ration alimentaire de l'animal, constitué à titre d'exemple par un mélange de céréales, de protéines et de matières grasses d'origine animale et/ou végétale.

Les bases nutritionnelles pour animaux sont adaptées à l'alimentation de ces animaux et sont bien connues de l'homme du métier. Habituellement, ces bases nutritionnelles comprennent par exemple du maïs, du blé, du pois et du soja. Ces bases nutritionnelles sont adaptées aux besoins des différentes espèces animales auxquelles elles sont destinées. Ces bases nutritionnelles peuvent déjà contenir des additifs nutritionnels comme des vitamines, des sels minéraux et des acides aminés.

Dans un mode de réalisation préféré, l'invention concerne des aliments pour animaux monogastriques et notamment pour les volailles et les porcs. Les volailles comprennent notamment les poules pondeuses, les poulets de chair, les dindes et les canards. Les porcs comprennent notamment les porcs croissance et finition ainsi que les porcelets.

### Description des figures

Figure 1 : Détermination du pH optimum de l'enzyme ABFB-1 dans une série de tampon Mc IIvaine (pH 2,2 à 8) à 40 °C en présence de 5 mM de PNPAF.
Figure 2 : Détermination de la température optimale de l'enzyme ABFB-1 à son pH optimum en présence de 5 mM de PNPAF. PNPAF.

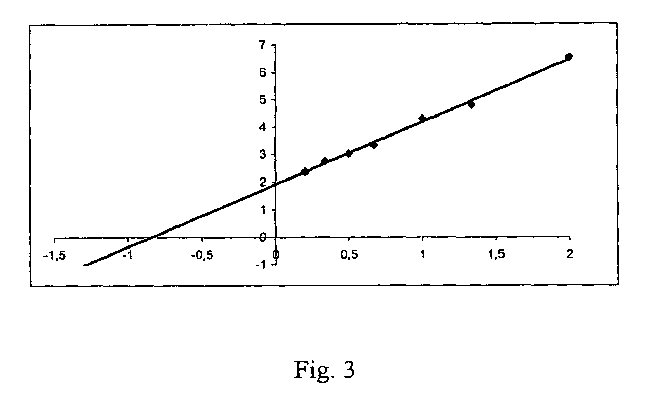

Figure 3 : Détermination des constantes cinétiques *Kₘ* et *Vₘ* (1/Vi = f (1/S) pour l'ABFB-1 pour une gamme de PNPAF allant de 0,5 mM à 5 mM à pH 3.4 et 60°C.
Figure 4 : Valeurs d'expression différentielle quantitatives relatives des gènes *abfB-1* et *abfB-2* en fonction des conditions de croissance de *P. funiculosum.*

### Exemples

### Mise au point du dosage de l'activité L-arabinofuranosidase B

L'activité L-arabinofuranosidase a été mesurée à partir d'une culture de *P. funiculosum* sur le milieu M2 avec un ajout mixte composé de 0,15% de Provasoy et 0,3% de cellulose au bout de 40h. Des prélèvements ont été réalisés à 48h et 72h de culture. La culture a été réalisée en erlen de 200 mL avec un volume utile de 50 mL. L'activité a été déterminée par hydrolyse de 5mM de para-nitrophényl-(-L-arabinofuranoside (PNPAF) dans un tampon 50 mM sodium acétate, pH 5. 50 µL de surnageant de culture ont été incubés avec 250µL de substrat préchauffé à 50°C pendant 15 min. La réaction a été stoppée par ajout de 500µL de 0,5 M NaOH. La libération du p-nitrophényl (PNP) est mesurée à 405 nm avec un coefficient d'extinction molaire de 17000 M-1.cm-1. Une unité enzymatique est définie comme la quantité d'enzyme qui hydrolyse 1 µmol de PNPAF par min dans les conditions décrites précédement. Pour la culture de *P. funiculosum* nous avons obtenus 20 mU.mL-1 au bout de 48h et 112 mU.mL-1 après 72h de culture. Ces résultats sont en accord avec la littérature, en effet pour *Aspergillus niger* des activités de l'ordre de 100 à 600 mU.mL-1 ont été trouvées suivant l'inducteur utilisé dans la culture.

### Clonage de l'ORF abfB de P. funiculosum chez Saccharomyces cerevisiae

A partir de l'ADN génomique de *P. funiculosum,* le gène *abfB* a été amplifié par PCR à l'aide du couple d'amorces (Hind III-abfB /Xba I-abfB) dans les conditions suivantes (94°C 30 sec ; 62°C 30 sec; 1 min 30 sec à 72°C) pendant 30 cycles. Le produit PCR a été cloné dans un vecteur commercial pGEM-T(tm) easy.

Séquence du couple d'amorce PCR
Xba I- abfB-1 : > 5'-TCTAGAATGTTTCCAAGAATAAAACCAG-3' <
Hind III- abfB-1: > 5'-AAGCTTTCATGCAAAGGCAGTCT- 3' <

Le fragment Hind III/Xba I de 1534 bp a été excisé du vecteur pGEM-T et sous cloné aux sites Hind III/Xba I dans un vecteur navette pJL 52 (plac195-PGK/CYC1). Pour l'expression hétérologue le gène *abfB* se trouve donc sous la dépendance du promoteur constitutif *PGK* du gène codant pour la phospho-glycérate kinase *(S*. *cerevisiae)* et du terminateur *CYC1 (S. cerevisiae)* du gène codant pour une activité cytochrome C oxydase. La nouvelle cassette d'expression est dénommée pOT-01.

La souche *S.cerevisiae* JF #1194 (CEN.PK113-5D), clone issu de la souche CEN.PK 122 portant l'auxotrophie *ura 3-52,* à été transformée (méthode au lithium acétate/ choc thermique) par le vecteur d'expression pOT-O1. Les souches transformantes ont été sélectionnées par complémentation phénotypique sur boites sélectives sans uracile (marqueur *URA3*).

Six transformants ont été sélectionnés pour tester la présence d'une activité arabinofuranosidase B dans le surnageant de culture. Les transformants ont été cultivés dans 50 mL de milieu YNB sans uracile (hormis la souche contrôle sauvage) pendant 24 heures. L'activité arabinofuranosidase a été dosée sur les surnageants de culture à l'aide de la méthode décrite dans le paragraphe précédent.

### Détermination du pH optimum

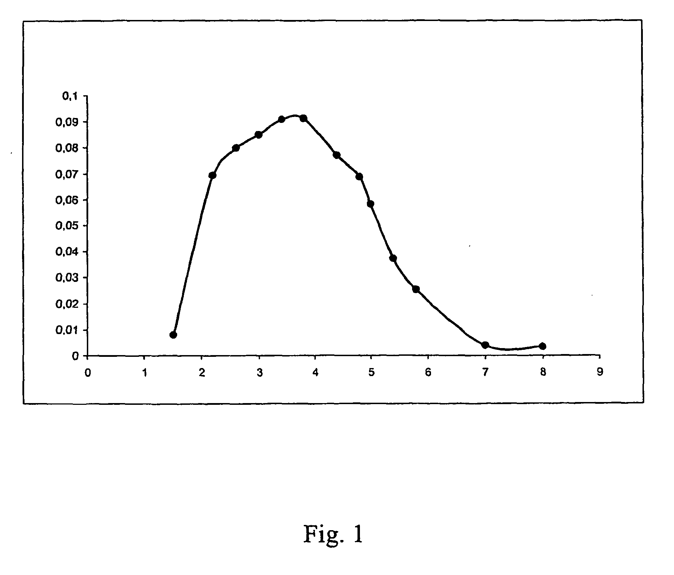

Le gène *abfB-1* codant pour une activité arabinofuranosidase B issue de *P. funiculosum* a été cloné chez *S*. *cerevisiae.* Après vérification de la présence d'une activité arabinofuranosidase B chez plusieurs transformants, un transformant a été choisi et l'activité ABFB à été dosée sur le surnageant de culture après 24 h de croissance. Les cultures ont été réalisées en erlen de 200 mL (Vu 50 mL). L'activité a été déterminée en présence de 5 mM de p-nitrophényl-α-L-arabinofuranoside (PNPAF) dans une série de tampon Mc Ilvaine (pH 2,2 à 8,0). 80µL de surnageant de culture ont été incubés avec 320 µL de substrat préchauffé à 40°C pendant 10 min. La réaction a été stoppée par ajout de 1 mL de Na₂CO₃ 1 M. La libération du p-nitrophényl est mesurée à 405 nm⁻¹. Une unité enzymatique est définie comme la quantité d'enzyme qui hydrolyse 1 µmol de PNPAF par min dans les conditions définies ci-dessus. La courbe d'activité est représentée à la **figure 1**. Pour l'ABFB-1, l'optimum d'activité se situe à pH 3,4 et l'enzyme conserve 65% d'activité à pH 5.

### Détermination de la température optimale

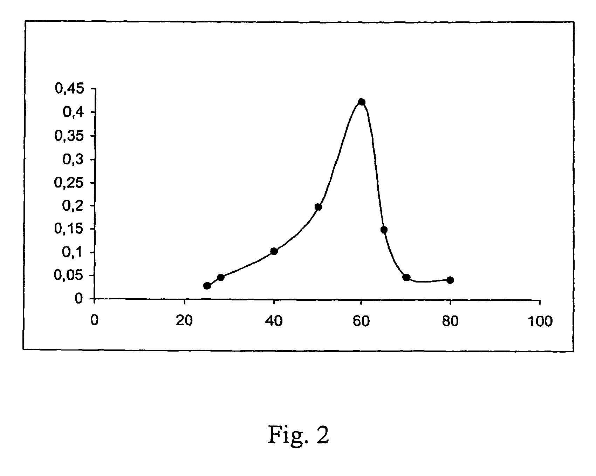

Selon le même protocole nous avons déterminé la température optimale d'activité de ABFB-1. L'enzyme a été incubée 10 min à chacune des températures dans un tampon Mc Ilvaine au pH 3,4. La courbe d'activité est présentée à la **figure 2**. L'ABFB-1 de *P. funiculosum* présente un optimum d'activité à 60°C. L'ABFB-1 présente donc un optimum de température supérieur aux ABFB décrites. En se plaçant aux pH et aux températures optimales de l'enzyme ABFB-1 (pH 3,4 et 60°C), nous remarquons que l'activité pour l'ABFB-1 est 4 fois supérieure à l'activité déterminée dans un tampon acétate pH 5 et 40°C (424 mU vs 102 mU).

### Détermination de Kₘ et Vₘ

Les constantes cinétiques (*Kₘ* et *Vₘ*) de l'ABFB-1 ont été déterminées par mesure de l'hydrolyse du PNPAF au cours du temps, dans les conditions optimales déterminées précédemment.

Les gammes de concentration en substrat (PNPAF) ont été établies entre 0,5 et 5 mM dans un tampon pH 3,4. La cinétique d'hydrolyse a été suivie pendant 10 minutes à 60°C. Les résultats ont été traités selon la méthode des doubles inverses (Lineweark et Burk) et présentés dans la

### figure 3.

La valeur de Km est de 1 mM pour l'ABFB-1. Par comparaison dans la littérature les valeurs de Km pour ce type d'enzyme varie de 0,05 à 1,2 mM selon le genre et l'espèce fongique étudié. L'ABFB-1 présente une vitesse d'hydrolyse maximale (*Vₘ*) de 521 mol PNPAF/mole d'enzyme/min dans les conditions décrites précédement.

### Détermination du poids moléculaire de l'enzyme ABFB-1

Afin de déterminer le poids moléculaire de l'enzyme ABFB-1, le surnageant de culture, issu de la croissance en milieu minimum d'un mutant (*S.cerevisiae*), a été concentré 200 fois, dénaturé par ébullition à 100°C pendant 5 min, puis déposé dans un gel de polyacrylamide-SDS.

On observe que la quantité de protéines extracellulaires est extrêmement faible dans la souche sauvage. Pour les mutants, l'enzyme ABFB-1 est sécrétée dans le surnageant de culture. Elle est majoritaire par rapport au niveau basal des protéines extracellulaires de *S. cerevisiae.*

La détermination du poids moléculaire a été effectuée à l'aide du marqueur de taille SeeBlue (Invitrogen). Les résultats sont présentés dans le tableau 1.

**Tableau 1 : Poids Moléculaire ABFB-1 en KDa**

| | **PM prédit** | **PM estimé sur gel** |
|---|---|---|
| **ABFB-1** | **53** | **65** |

Nous avons comparé le poids moléculaire prédit par l'algorithme de Vector NTi et le poids obtenu par migration électrophorétique dans un gel dénaturant SDS-PAGE. Nous observons une sur estimation du poids moléculaire de l'enzyme dans le gel SDS-PAGE. Une forte glycosylation de l'enzyme est en effet suggérée par la visualisation sur gel d'une bande électrophorétique diffuse (O et N glycosilations). Ces glycosylations surviennent lors de la maturation des protéines dans l'organisme d'expression.

### Analyse du profil d'expression du gène abfB-1 chez Penicillium funiculosum

*Penicillium funiculosum* possède deux gènes codant pour des α-L-arabinofuranosidase B : les gènes *abfB-1* et *abfB-2.* Les profils d'expression de ces gènes dans différentes conditions de culture de P.funiculosum ont été comparés.

*P. funiculosum* a été cultivé dans des conditions d'induction d'enzymes celluloytiques et hémi-cellulolytiques (milieu de croissance industriel de type M2) et dans des conditions de non production (milieu minimum glucose MO). Après 40h de croissance les cultures ont été arrêtées, le mycélium a été récupéré, et les ARN totaux ont été extraits. La quantité et la qualité des ARN ont été appréciées par mesure de l'absorbance à 260nm et à 280 nm (Ratio 260/280 > 1,8). Le niveau de transcrits codant pour les activités arabinofuranosidases de type B (ABFB-1 et ABFB-2) ont été quantifiés dans chacune des deux conditions (MO et M2) par PCR quantitative en temps réel.

Le gène codant pour la tubuline *(tub*-1*)* de *P. funiculosum* a été utilisé comme contrôle dans les deux conditions. Ce gène code pour une protéine de structure indispensable pour l'intégrité de la cellule. Ce gène est utilisé de façon courante comme gène de référence, car il présente un niveau d'expression constant quelque soit la condition de culture utilisée (ubiquitaire).

Des amorces spécifiques pour la PCR quantitative ont été dessinées pour chacun des gènes (*abfB*-1, *abfB*-2, et *tub*-1). Pour les deux conditions de croissance (M0 et M2), 2 µg d'ARN totaux ont été rétro-transcripts. Une série de dilution des ADN complémentaires issus de la rétro-transcription a été réalisée afin de déterminer les conditions optimales d'amplification des gènes cibles (contraintes de la méthode de PCR quantitative et pour l'efficacité des ces couples d'amorces).

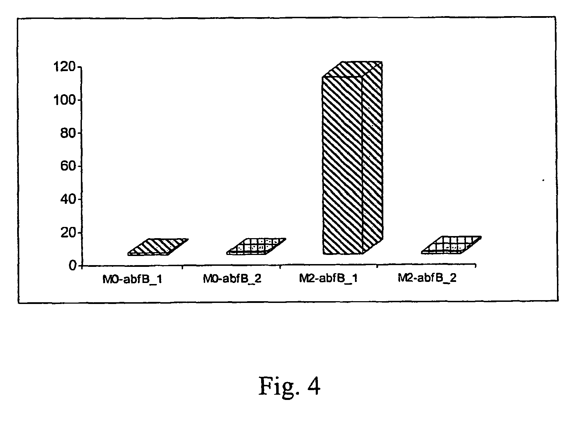

Les résultats normalisés sont présentés dans le tableau 2 et la **figure 4****.**

**Tableau 2 : Valeurs d'expression différentielle des gènes abfB-1 et abfB-2 en fonction des conditions de croissance de P. funiculosum**

| | **M0** | **M2** |
|---|---|---|
| **abfb 1** | 1 | 107 |
| **abfb 2** | 1 | 1,27 |

Les régulations transcriptionnelles des gènes codant pour des activités cellulolytiques et hémi-cellulolytiques ont été décrites. L'expression de ces gènes est fortement soumise à la nature et/ou à la complexité de la source de carbone et d'azote sur laquelle le microorganisme est cultivé. Il a été rapporté une forte répression transcriptionnelle de ces gènes en présence de glucose. Cette régulation est effectuée par l'intermédiaire d'une protéine de répression catabolique CreA qui se fixe spécifiquement sur le promoteur de ces gènes et bloque leur transcription. Dans notre expérience de quantification par PCR des messagers *abfB*-1 et *abfB*-2*,* que le niveau d'expression de ces 2 gènes dans la condition glucose (M0) est très faible. Ceci est en accord avec la littérature puisqu'il a été montré que ces gènes présentent un niveau basal d'expression même dans des conditions non favorables (absence de substrats cellulolytiques et / ou hémi cellulolytiques). Les résultats obtenus pour la condition M0 sont en accord avec la littérature. En ce qui concerne l'expression du gène *abfB*-1, nous constatons un facteur d'induction de 107 fois supérieur au niveau basal obtenu dans la condition M0, tandis que le gène abfB-2 n'est pas surexprimé.

## Revendications

1. Polypeptide adapté à l'utilisation en nutrition animale **caractérisé en ce qu'**il comprend un polypeptide choisi parmi les polypeptides suivants:
- le polypeptide de la SEQ ID No. 2 ,
- le polypeptide **mature** dont la séquence est comprise entre la position 28 et la position 507 de la SEQ ID No. 2,

2. Polynucléotide codant pour une activité α-L-arabinofuranosidase B **caractérisé en ce qu'**il est choisi parmi les polynucléotides suivants :
- le polynucléotide dont la séquence est comprise entre la position 845 et la position 2368 de la SEQ ID No. 1,
- le polynucléotide dont la séquence est comprise entre la position 926 et la position 2368 de la SEQ ID No. 1

3. Polynucléotide **caractérisé en ce qu'**il a la séquence de la SEQ ID No. 1 ou la séquence complémentaire à la SEQ ID No. 1.

4. Cassette d'expression **caractérisée en ce qu'**elle comprend dans le sens de la transcription:
- un promoteur fonctionnel dans un organisme hôte;
- un polynucléotide selon la revendication 2; et
- une séquence terminatrice dans le même organisme hôte.

5. Vecteur comprenant un polynucléotide selon l'une des revendications 2-3 et/ou une cassette d'expression selon la revendication 4.

6. Organisme hôte transformé avec un polynucléotide selon l'une des revendications 2-3, une cassette d'expression selon la revendication 4 et/ou un vecteur selon la revendication 5.

7. Organisme hôte selon la revendication 6 **caractérisé en ce que** l'organisme hôte est choisi parmi les levures et les champignons filamenteux.

8. Organisme hôte selon la revendication 7 **caractérisé en ce qu'**il s'agit d'une souche de *Penicillium funiculosum.*

9. Additif nutritionnel pour animaux **caractérisé en ce qu'**il comprend un polypeptide selon la revendication 1.

10. Additif nutritionnel pour animaux **caractérisé en ce qu'**il comprend un organisme hôte selon l'une des revendications 6-8 et/ou un moût de fermentation d'un organisme hôte selon l'une des revendications 6-8.

11. Additif nutritionnel pour animaux selon l'une des revendications 9-10 **caractérisé en ce qu'**il se présente sous forme liquide ou sous forme de poudre.

12. Aliment pour animaux **caractérisé en ce qu'**il comprend une base nutritionnelle pour animaux et un additif nutritionnel pour animaux selon l'une des revendications 9-11.

13. Utilisation d'un polypeptide selon la revendication 1 ou d'un organisme hôte selon l'une des revendications 6-8 pour la fabrication d'un additif nutritionnel pour animaux ou d'un aliment pour animaux.

14. Utilisation d'un polypeptide selon la revendication 1 ou d'un organisme hôte selon l'une des revendications 6-8 pour l'hydrolyse des liaisons α-L-arabinofuranosyl des composés arabinofuranosyl-oligosaccharides

## Claims

1. A polypeptide adapted for use in animal nutrition, **characterized in that** it comprises a polypeptide chosen from among the following polypeptides:
- the polypeptide of SEQ ID No. 2;
- the **mature** polypeptide whose sequence is between position 28 and position 507 of SEQ.ID No. 2.

2. A polynucleotide encoding an α-L-arabino-furanosidase B activity, **characterized in that** it is chosen from among the following polynucleotides:
- the polynucleotide whose sequence is between position 845 and position 2368 of SEQ ID No. 1;
- the polynucleotide whose sequence is between position **926** and position 2368 of SEQ ID No. 1.

3. A polynucleotide **characterized in that** it has the sequence of SEQ ID No. 1 or the complementary sequence for SEQ ID No.1.

4. An expression cassette **characterized in that** it comprises in the direction of transcription:
- a functional promoter in a host organism;
- a polynucleotide according to claim 2; and
- a terminator sequence in the same host organism.

5. A vector comprising a polynucleotide according to one of claims 2-3 and/or an expression cassette according to claim 4.

6. A host organism transformed with a polynucleotide according to anyone of claims 2-3, an expression cassette according to claim 4 and/or a vector according to claim 5.

7. The host organism according to claim 6, **characterized in that** the host organism is chosen from among yeasts and filamentous fungi.

8. The host organism according to claim 7, **characterized in that** it is a strain of *Penicillium funiculosum.*

9. A nutritional additive for animals **characterized in that** it comprises a polypeptide according to claim 1.

10. A nutritional additive for animals **characterized in that** it comprises a host organism according to one of claims 6-8 and/or fermentation must of a host organism according to one of claims 6-8.

11. A nutritional additive for animals according to one of claims 9-10, **characterized in that** it is in liquid form or power form.

12. Animal feed **characterized in that** it comprises a nutritional base for animals and a nutritional additive for animals according to one of claims 9-11.

13. Use of a polypeptide according to claim 1 or of a host organism according to one of claims 6-8 to produce a nutritional additive for animals or an animal feed.

14. Use of a polypeptide according to claim 1 or of a host organism according to one of claims 6-8 to hydrolyse α-L-arabinofuranosyl bonds of arabinofuranosyl-oligosaccharide compounds.

## Patentansprüche

1. Polypeptid, das für die Verwendung in der Tierernährung geeignet ist, **dadurch gekennzeichnet, dass** es ein Polypeptid umfasst, das aus den folgenden Polypeptiden ausgewählt ist:
- dem Polypeptid der SEQ ID No. 2,
- dem reifen Polypeptid, dessen Sequenz zwischen der Position 28 und der Position 507 der SEQ ID No. 2 liegt.

2. Polynukleotid, das für eine α-L-arabinofuranosidase B Aktivität codiert, **dadurch gekennzeichnet, dass** es aus den folgenden Polynukleotiden ausgewählt ist:
- dem Polynukleotid, dessen Sequenz zwischen der Position 845 und der Position 2368 der SEQ ID No. 1 liegt,
- dem Polynukleotid, dessen Sequenz zwischen der Position 926 und der Position 2368 der SEQ ID No. 1 liegt.

3. Polynukleotid, **dadurch gekennzeichnet, dass** es die Sequenz der SEQ ID No. 1 oder die zu der SEQ ID No. 1 komplementäre Sequenz hat.

4. Expressionskassette, **dadurch gekennzeichnet, dass** sie in Richtung der Transkription umfasst:
- einen Funktionspromotor in einem Wirtsorganismus,
- ein Polynukleotid nach Anspruch 2 und
- eine terminale Sequenz in demselben Wirtsorganismus.

5. Vektor, der ein Polynukleotid nach einem der Ansprüche 2 bis 3 und/oder eine Expressionskassette nach Anspruch 4 umfasst.

6. Wirtsorganismus, transformiert mit einem Polynukleotid nach einem der Ansprüche 2 bis 3, eine Expressionskassette nach Anspruch 4 und/oder ein Vektor nach Anspruch 5.

7. Wirtsorganismus nach Anspruch 6, **dadurch gekennzeichnet, dass** der Wirtsorganismus aus den Hefen und den Fadenpilzen ausgewählt ist.

8. Wirtsorganismus nach Anspruch 7, **dadurch gekennzeichnet, dass** es sich um einen Stamm von *Penicillium funiculosum* handelt.

9. Nahrungsergänzung für Tiere, **dadurch gekennzeichnet, dass** sie ein Polypeptid nach Anspruch 1 umfasst.

10. Nahrungsergänzung für Tiere, **dadurch gekennzeichnet, dass** sie einen Wirtsorganismus nach einem der Ansprüche 6 bis 8 und/oder einen Gärungsmost eines Wirtsorganismus nach einem der Ansprüche 6 bis 8 umfasst.

11. Nahrungsergänzung für Tiere nach einem der Ansprüche 9 bis 10, **dadurch gekennzeichnet, dass** sie sich in flüssiger Form oder in Pulverform darstellt.

12. Tiernahrung, **dadurch gekennzeichnet, dass** sie eine Ernährungsbasis für Tiere und eine Nahrungsergänzung für Tiere nach einem der Ansprüche 9 bis 11 umfasst.

13. Verwendung eines Polypeptids nach Anspruch 1 oder eines Wirtsorganismus nach einem der Ansprüche 6 bis 8 für die Herstellung einer Nahrungsergänzung für Tiere oder einer Tiernahrung.

14. Verwendung eines Polypeptids nach Anspruch 1 oder eines Wirtsorganismus nach einem der Ansprüche 6 bis 8 für die Hydrolyse der α-L-arabinofuranosyl-Bindungen der Arabinofuranosyl-Oligosaccharid-Verbindungen.


### LISTE DE SEQUENCES

<110> ADISSEO FRANCE SAS
<120> Gène abfb-1 de *Penicillium funiculosum*
<130> KH/SG/BR048786
<140>
   <141>
<160> 4
<170> PatentIn Ver. 2.1
<210> 1
   <211> 3107
   <212> ADN
   <213> Penicillium funiculosum
<220>
   <221> promoter
   <222> (1)..(844)
<220>
   <221> misc_feature
   <222> (212) .. (216)
   <223> Site creA
<220>
   <221> misc_feature
   <222> (403)..(408)
   <223> Site pacC
<220>
   <221> misc_feature
   <222> (559)..(564)
   <223> Site creA
<220>
   <221> misc_feature
   <222> (616)..(620)
   <223> Site AlcR
<220>
   <221> TATA_signal
   <222> (739)..(742)
<220>
   <221> CDS
   <222> (845)..(2368)
<220>
   <221> terminator
   <222> (2369)..(3107)
<220>
   <221> polyA_signal
   <222> (2460)..(2465)
<220>
   <221> sig_peptide
   <222> (845)..(926)
<400> 1
   agtgattgat cattacagtc ggcaaaaacg gtccgtttat ccccggatga gcatgcgcga 60
   agttgattag attgcaatcg gataaattgc attgcatgac tacgtgcctc ggcaaagatg 120
   gacctcatcg agtgtatgag ctcggcattc aagtttaaag agtaatttaa ccttggggat 180
   caaggacaag gctttttaag cttatatgcg aggatccggg gccccagggt gccatgactc 240
   ctcaacattc tgtttcaatt ggccaattgc ccctcctgag accctataac acgcggcgtt 300
   ttcttctcct cttgggcaag cttagttgtg ctcaaatcgc tgtacctgaa ttaggcaggt 360
   actcggcact cacacctatg atatcgtggc ctggtctaac ttgccaggaa atcggaattt 420
   tcagactctt tttcttgccg atttgggatc aatcctaatt ttagttgcat caaggtaata 480
   aacggaacac tttgttccac ctctgatgct cgtgagaaat agccttacac gcgtcacaag 540
   gcttttgttt atgcaacgct ggagagggac agcatcgttt ttctccacgt tttttcaaat 600
   tcttctgaat gcaccccgca taacggtaat tagcattagc attgtatgca tatcgagttt 660
   gtaattattg gagggatttc atgccacctg agtaaaatga gccggtctga atctggttct 720
   cggtagtttg aaaatgatta taaaagatag gtttcggctt gaaacgtgct ctgcttcagt 780
   agtcgattaa gcctctcatc gatcatctta tagtcacagg cgtgctcgtc tagcaaaagt 840
   catc atg ttt cca aga ata aaa cca gaa cgc acc agc ctg ttc gcc ctt  889
        Met Phe Pro Arg Ile Lys Pro Glu Arg Thr Ser Leu Phe Ala Leu
        1               5                   10                  15
   ggc ctt ctt gcc tcg agc tcc ctt gtc acc gca act ggg cct tgc gat   937
   Gly Leu Leu Ala Ser Ser Ser Leu Val Thr Ala Thr Gly Pro Cys Asp
                   20                  25                  30
   atc tac tcc tcc ggc ggt acg cct tgc gtt gcc gcg cac agc acc acc   985
   Ile Tyr Ser Ser Gly Gly Thr Pro Cys Val Ala Ala His Ser Thr Thr
               35                  40                  45
   cgt gcc ctt tat gcc tct tac agc ggc gca ctc tac cag gtc aag cgg   1033
   Arg Ala Leu Tyr Ala Ser Tyr Ser Gly Ala Leu Tyr Gln Val Lys Arg
           50                  55                  60
   ggc tcc gac ggt gct aca acc acc atc tcg ccg ctt tcc gca ggt ggt   1081
   Gly Ser Asp Gly Ala Thr Thr Thr Ile Ser Pro Leu Ser Ala Gly Gly
       65                  70                  75
   gtc gcc aac gct gcc gcc caa gat acc ttc tgt gcc aac acg acc tgc   1129
   Val Ala Asn Ala Ala Ala Gln Asp Thr Phe Cys Ala Asn Thr Thr Cys
   80                  85                  90                  95
   ctc atc acg atc atc tac gac caa tcc ggc cgc ggt aac cat ctc acc   1177
   Leu Ile Thr Ile Ile Tyr Asp Gln Ser Gly Arg Gly Asn His Leu Thr
                   100                 105                 110
   cag gct ccc ccc ggt ggc ttc gat ggc cct gac gtc aat ggc tat gat   1225
   Gln Ala Pro Pro Gly Gly Phe Asp Gly Pro Asp Val Asn Gly Tyr Asp
               115                 120                 125
   aac ttg gct ggc gcg att ggc gca ccc gtc act ctg aac ggt cag aag    1273
   Asn Leu Ala Gly Ala Ile Gly Ala Pro Val Thr Leu Asn Gly Gln Lys
           130                 135                 140
   gcg tac ggt gtc ttc atc tca cct ggt acc ggc tac cgt aac aac gct    1321
   Ala Tyr Gly Val Phe Ile Ser Pro Gly Thr Gly Tyr Arg Asn Asn Ala
       145                 150                 155
   gcc agc ggc aca gct acc ggt gac gca gcg gag ggc atg tat gca gtg    1369
   Ala Ser Gly Thr Ala Thr Gly Asp Ala Ala Glu Gly Met Tyr Ala Val
   160                 165                 170                 175
   ctc gac gga act cac tac aac ggc gcg tgc tgc ttc gat tat ggc aat    1417
   Leu Asp Gly Thr His Tyr Asn Gly Ala Cys Cys Phe Asp Tyr Gly Asn
                   180                 185                 190
   gcc gag acc agc agc act gat act ggc aat ggc cac atg gag gct atc    1465
   Ala Glu Thr Ser Ser Thr Asp Thr Gly Asn Gly His Met Glu Ala Ile
               195                 200                 205
   tac tat ggt gac gcc acc tac tgg gga agt ggc tcc ggc agc ggg cca    1513
   Tyr Tyr Gly Asp Ala Thr Tyr Trp Gly Ser Gly Ser Gly Ser Gly Pro
           210                 215                 220
   tgg gtc atg gct gat ctt gag aac ggt ctg ttc tct ggt gag agc act    1561
   Trp Val Met Ala Asp Leu Glu Asn Gly Leu Phe Ser Gly Glu Ser Thr
       225                 230                 235
   ggc gtg aac tct gcc gac ccg tct ctc tct tac cgc ttc gtc act gcg    1609
   Gly Val Asn Ser Ala Asp Pro Ser Leu Ser Tyr Arg Phe Val Thr Ala
   240                 245                 250                 255
   gtc gtc aag ggt gag cca aat ttc tgg gct att cgc ggt ggt aac gct    1657
   Val Val Lys Gly Glu Pro Asn Phe Trp Ala Ile Arg Gly Gly Asn Ala
                   260                 265                 270
   gca tcc ggt tct ttg tcc acg tac tac agt ggg gtg cgc cca caa gta    1705
   Ala Ser Gly Ser Leu Ser Thr Tyr Tyr Ser Gly Val Arg Pro Gln Val
               275                 280                 285
   tct ggc tat tac ccc atg cac aaa gaa ggt gcc atc att ctc ggc att    1753
   Ser Gly Tyr Tyr Pro Met His Lys Glu Gly Ala Ile Ile Leu Gly Ile
           290                 295                 300
   ggt ggt gat aac agc aac ggc gcc cag ggc acg ttc tac gaa ggt gtc    1801
   Gly Gly Asp Asn Ser Asn Gly Ala Gln Gly Thr Phe Tyr Glu Gly Val
       305                 310                 315
   atg acc tcc gga tat cca aca gac gcc acc gag aac tca gtg cag gct    1849
   Met Thr Ser Gly Tyr Pro Thr Asp Ala Thr Glu Asn Ser Val Gln Ala
   320                 325                 330                 335
   aac atc gta gct gcc aaa tac gct acc acg tcg ttg acc agt ggt ccg    1897
   Asn Ile Val Ala Ala Lys Tyr Ala Thr Thr Ser Leu Thr Ser Gly Pro
                   340                 345                 350
   gca ctc aca gtc ggc tcc gct att tcg ttg cat gtc acc act gcc gga    1945
   Ala Leu Thr Val Gly Ser Ala Ile Ser Leu His Val Thr Thr Ala Gly
               355                 360                 365
   tac acg aca cgc tat atc gca cac aac gac acc acc gtc aac acc cag    1993
   Tyr Thr Thr Arg Tyr Ile Ala His Asn Asp Thr Thr Val Asn Thr Gln
           370                   375                   380
   gtc gtc tca tcg tcc agc tct acc act ctc aaa gag caa gct agc tgg    2041
   Val Val Ser Ser Ser Ser Ser Thr Thr Leu Lys Glu Gln Ala Ser Trp
       385                 390                 395
   acg gtt cgc acc ggt ctt ggc aac agc gcc tgt ttc tcg ttt gag tcc    2089
   Thr Val Arg Thr Gly Leu Gly Asn Ser Ala Cys Phe Ser Phe Glu Ser
   400                 405                 410                 415
   gtt gac acc ccc ggc agc tac att cgt cac tac aac ttc gag ctc ctg    2137
   Val Asp Thr Pro Gly Ser Tyr Ile Arg His Tyr Asn Phe Glu Leu Leu
                   420                 425                 430
   ctc aat gct aat gat ggc acc aag cag ttc cat gaa gat gcc aca ttc    2185
   Leu Asn Ala Asn Asp Gly Thr Lys Gln Phe His Glu Asp Ala Thr Phe
               435                 440                 445
   tgc ccg cag tct ggc ctt agc ggc acg ggt acc tcg ctt cga agc tgg    2233
   Cys Pro Gln Ser Gly Leu Ser Gly Thr Gly Thr Ser Leu Arg Ser Trp
           450                 455                 460
   agc tat ccc acc cgc tat ttc cga cac tat aat aac gta ttg tat gct    2281
   Ser Tyr Pro Thr Arg Tyr Phe Arg His Tyr Asn Asn Val Leu Tyr Ala
       465                 470                 475
   gcc agc aac ggc ggc gtg caa acg ttt gat gcc acc gct tcc ttc aac    2329
   Ala Ser Asn Gly Gly Val Gln Thr Phe Asp Ala Thr Ala Ser Phe Asn
   480                 485                 490                 495
   gcc gat gtg acc ttc ttg gtc gag act gcc ttt gca tga ttcttgacct     2378
   Ala Asp Val Thr Phe Leu Val Glu Thr Ala Phe Ala
                   500                 505
   agcctcgtga aggtctaggt gtatggtaga accttgttag tgtatccttg gcacgattcg 2438
   ttttaaatat aggcagattt aaataaactg atgattacaa agaagacttg gcttacctgt 2498
   tagtaactat ctcaaagatg aagttgctgt ttgttgtagg aagatgccct gcatgcaaac 2558
   aaaacaaact tcttgggcga tgaaggtcac gtgactcgta gccggtattg attacctgtt 2618
   tgggaaacat tcacgccaac aactccgagg caatagtcag gcttgcaacg ataccaaaga 2678
   gaaagagaaa cggaggtttg tataagagaa atattgtcta gcaaactgac agtgactcat 2738
   tccaccaaaa tgtatgtatg agttggccat gctctaagag ttcctgtagg gcaaccactg 2798
   gctctaattg gctgccgggc ggcttcctcc cacggacccg ccctccagat gatcgtagca 2858
   agttgccgcc gattaatttg ccagccacct tccctctttc tctcagggtc tgtctgtctg 2918
   tgttcgctgt tgttcctcct gccattcccc tctgctccca ttcggtctgt catcttttga 2978
   ataccctttg aatattcttc gcgcacactg agtgcgccca ttattgtatt attatcgact 3038
   ttgtcgtctc ttctcagact tttcgtgtgt ttttttgggt cttcgttatg ggtattccgt 3098
   aagttgatc                                                          3107
<210> 2
   <211> 507
   <212> PRT
   <213> Penicillium funiculosum
<400> 2
   Met Phe Pro Arg Ile Lys Pro Glu Arg Thr Ser Leu Phe Ala Leu Gly
   1 5 10 15
   Leu Leu Ala Ser Ser Ser Leu Val Thr Ala Thr Gly Pro Cys Asp Ile
   20 25 30
   Tyr Ser Ser Gly Gly Thr Pro Cys Val Ala Ala His Ser Thr Thr Arg
   35 40 45
   Ala Leu Tyr Ala Ser Tyr Ser Gly Ala Leu Tyr Gln Val Lys Arg Gly
   50 55 60
   Ser Asp Gly Ala Thr Thr Thr Ile Ser Pro Leu Ser Ala Gly Gly Val
   65 70 75 80
   Ala Asn Ala Ala Ala Gln Asp Thr Phe Cys Ala Asn Thr Thr Cys Leu
   85 90 95
   Ile Thr Ile Ile Tyr Asp Gln Ser Gly Arg Gly Asn His Leu Thr Gln
   100 105 110
   Ala Pro Pro Gly Gly Phe Asp Gly Pro Asp Val Asn Gly Tyr Asp Asn
   115 120 125
   Leu Ala Gly Ala Ile Gly Ala Pro Val Thr Leu Asn Gly Gln Lys Ala
   130 135 140
   Tyr Gly Val Phe Ile Ser Pro Gly Thr Gly Tyr Arg Asn Asn Ala Ala
   145 150 155 160
   Ser Gly Thr Ala Thr Gly Asp Ala Ala Glu Gly Met Tyr Ala Val Leu
   165 170 175
   Asp Gly Thr His Tyr Asn Gly Ala Cys Cys Phe Asp Tyr Gly Asn Ala
   180 185 190
   Glu Thr Ser Ser Thr Asp Thr Gly Asn Gly His Met Glu Ala Ile Tyr
   195 200 205
   Tyr Gly Asp Ala Thr Tyr Trp Gly Ser Gly Ser Gly Ser Gly Pro Trp
   210 215 220
   Val Met Ala Asp Leu Glu Asn Gly Leu Phe Ser Gly Glu Ser Thr Gly
   225 230 235 240
   Val Asn Ser Ala Asp Pro Ser Leu Ser Tyr Arg Phe Val Thr Ala Val
   245 250 255
   Val Lys Gly Glu Pro Asn Phe Trp Ala Ile Arg Gly Gly Asn Ala Ala
   260 265 270
   Ser Gly Ser Leu Ser Thr Tyr Tyr Ser Gly Val Arg Pro Gln Val Ser
   275 280 285
   Gly Tyr Tyr Pro Met His Lys Glu Gly Ala Ile Ile Leu Gly Ile Gly
   290 295 300
   Gly Asp Asn Ser Asn Gly Ala Gln Gly Thr Phe Tyr Glu Gly Val Met
   305 310 315 320
   Thr Ser Gly Tyr Pro Thr Asp Ala Thr Glu Asn Ser Val Gln Ala Asn
   325 330 335
   Ile Val Ala Ala Lys Tyr Ala Thr Thr Ser Leu Thr Ser Gly Pro Ala
   340 345 350
   Leu Thr Val Gly Ser Ala Ile Ser Leu His Val Thr Thr Ala Gly Tyr
   355 360 365
   Thr Thr Arg Tyr Ile Ala His Asn Asp Thr Thr Val Asn Thr Gln Val
   370 375 380
   Val Ser Ser Ser Ser Ser Thr Thr Leu Lys Glu Gln Ala Ser Trp Thr
   385 390 395 400
   Val Arg Thr Gly Leu Gly Asn Ser Ala Cys Phe Ser Phe Glu Ser Val
   405 410 415
   Asp Thr Pro Gly Ser Tyr Ile Arg His Tyr Asn Phe Glu Leu Leu Leu
   420 425 430
   Asn Ala Asn Asp Gly Thr Lys Gln Phe His Glu Asp Ala Thr Phe Cys
   435 440 445
   Pro Gln Ser Gly Leu Ser Gly Thr Gly Thr Ser Leu Arg Ser Trp Ser
   450 455 460
   Tyr Pro Thr Arg Tyr Phe Arg His Tyr Asn Asn Val Leu Tyr Ala Ala
   465                 470                 475                 480
   Ser Asn Gly Gly Val Gln Thr Phe Asp Ala Thr Ala Ser Phe Asn Ala
                   485                 490                 495
   Asp Val Thr Phe Leu Val Glu Thr Ala Phe Ala
               500                 505
<210> 3
   <211> 28
   <212> ADN
   <213> Séquence artificielle
<220>
   <223> Description de la séquence artificielle: amorce XbaI-abfB
<400> 3
   tctagaatgt ttccaagaat aaaaccag 28
<210> 4
   <211> 23
   <212> ADN
   <213> Séquence artificielle
<220>
   <223> Description de la séquence artificielle: Amorce HindIII-abfB
<400> 4
   aagctttcat gcaaaggcag tct 23